(19) **Europäisches Patentamt**

**European Patent Office**

**Office européen des brevets**

(11) **EP 0 838 194 B1**

(12) **EUROPEAN PATENT SPECIFICATION**

(45) Date of publication and mention of the grant of the patent:
**23.04.2003 Bulletin 2003/17**

(51) Int Cl.$^7$: **A61B 5/0205**, G06F 17/40

(21) Application number: **96115388.9**

(22) Date of filing: **25.09.1996**

(54) **Physical information monitor system having means for indicating amount of deviation of monitored information from normal information**

Physikalisches Informations-Überwachungssystem mit Anzeigevorrichtung für den Abweichungsbetrag einer überwachten Information vom Sollwert

Système de moniteur d'information physique comportant un dispositif d'affichage d'importance d'un défaut d'une information surveillée d'une information normale

(84) Designated Contracting States:
**DE FR GB**

(43) Date of publication of application:
**29.04.1998 Bulletin 1998/18**

(73) Proprietor: **Colin Corporation**
**Komaki-shi, Aichi-ken (JP)**

(72) Inventors:
- **Oka, Tohru, c/o Colin Corp.**
  **Komaki-shi, Aichi-ken (JP)**
- **Takakura, Makoto, c/o Colin Corp.**
  **Komaki-shi, Aichi-ken (JP)**

(74) Representative:
**Winter, Brandl, Fürniss, Hübner, Röss, Kaiser, Polte Partnerschaft**
**Patent- und Rechtsanwaltskanzlei**
**Alois-Steinecker-Strasse 22**
**85354 Freising (DE)**

(56) References cited:
| | |
|---|---|
| **DE-A- 4 219 588** | **FR-A- 2 534 132** |
| **GB-A- 2 281 781** | **US-A- 3 618 592** |
| **US-A- 5 355 889** | |

**Description**

**BACKGROUND OF THE INVENTION**

Field of the Invention

The present invention relates to a physical information monitor system for monitoring successively obtained physical information of living subjects.

Discussion of the Related Art

For monitoring the physical condition of a patient (living subject) in an operating room or ICU (Intensive Care Unit), there is proposed a physical information monitor system adapted to successively measure one or more physical parameters of the patient, such as blood pressure, pulse rate, blood oxygen saturation and temperature, and to generate an abnormality signal indicative of abnormality of any physical parameter when the physical parameter is outside a predetermined reference range.

In general, the conventional physical information monitor system as described above has abnormality determining means for determining whether the obtained physical information is outside a predetermined reference range. The abnormality determining means generates an abnormality signal when the obtained physical information is outside the reference range. The abnormality signal may be visually indicated by suitable characters given on a display, or by a flickering or colored display area in which the measured abnormal value is displayed, for instance. Alternatively, the abnormality may be indicated by a sound or voice. A device comprising the features of the preamble of claim 1 is disclosed in document GB-A-2 281 781.

In the conventional physical information monitor system, however, the abnormality determining means simply generates the abnormality signal when the obtained physical information of the subject is outside the reference range. That is, the system is not arranged to indicate the degree of deviation of the obtained physical information from the reference range. In the conventional arrangement, it is impossible to judge whether the physical information slightly deviates from the reference range or considerably deviates from the reference range. Thus, in the conventional monitor system, it is difficult to quickly recognize the degree of abnormality of the physical information, i.e., the degree of emergency to deal with the abnormal physical condition of the patient upon generation of the abnormality signal.

**SUMMARY OF THE INVENTION**

It is therefore an object of the present invention to provide a physical information monitor system which permits easier recognition of the degree of abnormality or emergency of physical condition of the living subject upon occurrence of abnormality of the physical information of the patient.

The above object may be attained according to a principle of the present invention which provides a physical information monitor system comprising: (a) physical information obtaining means for successively obtaining physical information of a living subject; (b) physical information memory means for storing the physical information which is previously obtained in a comparatively normal condition of the living subject; (c) abnormality determining means for determining that the physical information obtained by the physical information obtaining means is abnormal if the physical information is outside a predetermined reference range; (d) a display device for indicating the physical information obtained by the physical information obtaining means; and (e) indication control means for controlling the display device to indicate, in a same indication area of the display device, the physical information which has been determined to be abnormal by the abnormality determining means and the physical information previously obtained in the comparatively normal condition of the subject and stored by the physical information memory means.

In the monitor system constructed according to the present invention, the physical information memory means stores the physical information previously obtained in the comparatively normal condition of the subject. When the abnormality determining means determines that the physical information obtained by the obtaining means is outside the predetermined reference range, the indication control means activates the display device to indicate the physical information which has been determined to be abnormal by the determining means and the physical information which was previously obtained in the comparatively normal condition and stored in the memory means, in the same indication area of the display device.

Since the abnormal physical information and the normal physical information are indicated in the same indication area of the display device as described above, the present arrangement permits easier recognition of the degree of abnormality of the physical information, namely, the degree of emergency to deal with the abnormal physical condition of the subject, by comparing the difference between the normal and abnormal physical information indicated in the same indication area.

According to a first preferred form of the present invention, the physical information obtaining means obtains as the physical information a series of pulses of a pulse wave generated in synchronism with heartbeat of the subject, while the indication control means controls the display device to indicate a series of pulses of a normal pulse wave previously obtained in the comparatively normal condition of the subject and the series of pulses of the pulse wave obtained by the physical information obtaining means in an abnormal condition of the subject, in a same two-dimensional coordinate system in which time is taken along an abscissa while an amplitude of the series of pulses is taken along an ordinate. This arrangement makes it possible to accurately compare the difference in the amplitude between the series of pulses of the normal pulse wave and the series of pulses of the abnormal pulse wave, since they are indicated in the same two-dimensional coordinate system.

According to a second preferred form of the present invention, the physical information obtaining means obtains as the physical information a series of pulses of a pulse wave generated in synchronism with heartbeat of the subject, while the indication control means controls the display device to indicate, in a same two-dimensional coordinate system, a waveform of one of a series of pulses of a normal pulse wave previously obtained in a comparatively normal condition of the subject and a waveform of one of a series of pulses of the pulse wave obtained by the physical information obtaining means in an abnormal condition of the subject, such that phases of the waveforms of the normal pulse wave and the abnormal pulse wave coincide with each other, in the same two-dimensional coordinate system in which time is taken along an abscissa while an amplitude of the series of pulses is taken along an ordinate. In this arrangement, the configuration of the pulse waveform of the abnormal pulse wave can be easily compared with that of the pulse waveform of the normal pulse wave.

According to one advantageous arrangement of the above second preferred form of the present invention, the indication control means includes normalizing means for normalizing the waveforms of the normal pulse wave and the abnormal pulse wave, such that at least one of an amplitude and a wavelength of the abnormal pulse wave coincides with the corresponding one of the amplitude and wavelength of the normal pulse wave, the indication control means controlling the display device to indicate the waveforms of the normal pulse wave and the abnormal pulse wave which have been normalized by the normalizing means, such that the waveforms of the normal pulse wave and the abnormal pulse wave are superimposed on each other. This arrangement permits easier recognition of the difference between the configuration of the pulse waveform of the normal pulse wave and the configuration of the pulse waveform of the abnormal pulse wave.

In the above advantageous arrangement of the present invention, the indication control means includes calculating means for calculating an area difference index which quantitatively indicates a difference between an area defined by the waveform of the normal pulse wave and an area defined by the waveform of the abnormal pulse wave, the indication control means controlling the display device to indicate the area difference index calculated by the calculating means. In this arrangement, the difference between the waveform of the normal pulse wave and the waveform of the abnormal pulse wave can be quantitatively recognized.

## BRIEF DESCRIPTION OF THE DRAWINGS

The above and optional objects, features and advantages of the present invention will be better understood by reading the following detailed description of a presently preferred embodiment of the invention when considered in connection with the accompanying drawings in which:

Fig. 1 is a diagrammatic block diagram of a blood pressure monitor system according to one embodiment of the present invention;
Fig. 2 is a graph representing a relationship between a pulse wave magnitude $P_M$ and a monitored blood pressure MBP;
Fig. 3 is a block diagram schematically showing various functions of a control device used in the blood pressure monitor system of Fig. 1;
Fig. 4 is a flow chart representing a control routine executed by the control device in the blood pressure monitor system of Fig. 1;
Fig. 5 is a view showing one example of waveform indication as a result of an operation of the monitor system of Fig. 1 according to the control routine of the flow chart of Fig. 4; and
Fig. 6 is a view showing another example of waveform indication according to the control routine of Fig. 4.

## DETAILED DESCRIPTION OF THE PREFERRED EMBODIMENT

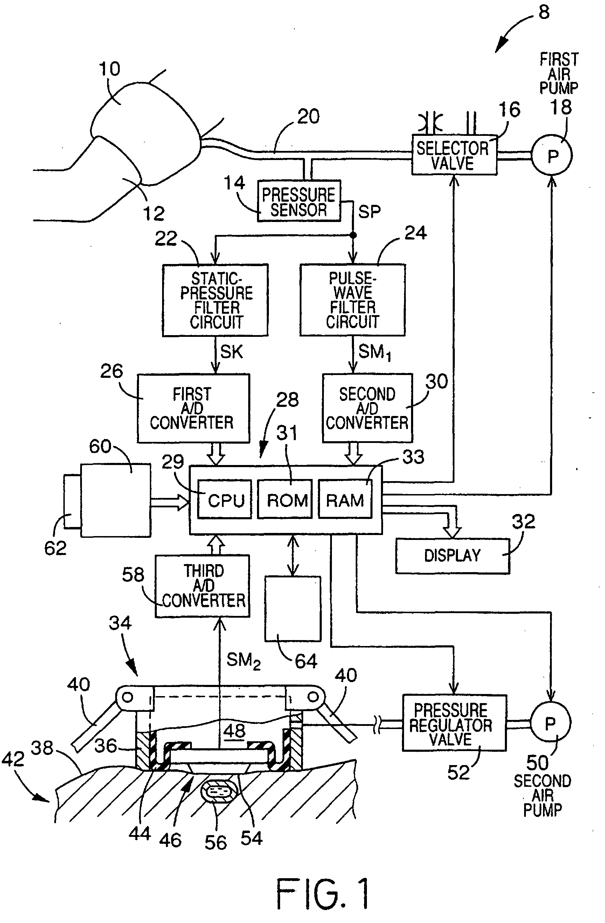

Referring first to Fig. 1, there is shown a blood pressure (BP) monitor system 8 constructed according to one embodiment of the present invention. In Fig. 1, the reference numeral 10 denotes an inflatable cuff constituted by an elongate fabric bag and a rubber bag accommodated in the elongate fabric bag. The cuff 10 is worn on a patient such that it is wound on an upper arm 12 of the patient, for example. A pressure sensor 14, a selector valve 16 and a first air pump 18 are connected to the cuff 10 via a conduit piping 20. The selector valve 16 is selectively placed in an inflation position, a slow-deflation position, and a rapid-deflation position. In the inflation position, the selector valve 16 permits pressurized air to be supplied from the first air pump 18 to the cuff 10. In the slow-deflation position, the selector valve 16 permits the pressurized air to be slowly discharged from the cuff 10. In the rapid-deflation position, the selector valve 16 permits the pressurized air to be rapidly discharged from the cuff 10.

The pressure sensor 14 detects an air pressure in the cuff 10 and supplies an electric signal SP representative of the detected pressure to a static-pressure filter circuit 22 and a pulse-wave filter circuit 24.

The static-pressure filter circuit 22 has a low-pass filter and transmits a static component of the signal SP as a cuff-pressure signal SK to a control device 28 via a first analog to digital (A/D) converter 26.

The pulse-wave filter circuit 24 has a band-pass filter and transmits an oscillating component of the signal SP as a cuff pulse wave signal $SM_1$ to the control device 28 via a second analog to digital (A/D) converter 30. The cuff pulse wave signal $SM_1$ is representative of a pulse wave, i.e., an oscillatory pressure wave which is produced from a brachial artery of the patient in synchronism with the heartbeat of the patient and transmitted to the cuff 10. In the present embodiment, the cuff 10, pressure sensor 14 and pulse-wave filter circuit 24 cooperate with each other to function as a pulse wave sensor for obtaining the blood pressure of the patient.

The control device 28 is constituted by a so-called microcomputer which includes a central processing unit (CPU) 29, a read only memory (ROM) 31, a random access memory (RAM) 33 and an input and output (I/O) port not shown. The CPU 29 performs signal processing operations according to control programs stored in the ROM 31 by utilizing a temporary data storage function of the RAM 33, and generates drive signals through the I/O port so as to control the selector valve 16 and first air pump 18.

The present monitor system 8 further includes a pulse wave probe 34. The probe 34 is set on a wrist 42 located downstream of the arterial vessel of either one of the two upper arms 12, on which the cuff 10 is worn or is not worn. The probe 34 includes a container-like housing 36 which is detachably set on a body surface 38 of the wrist 42 with a pair of bands 40, 40 fastened round the wrist 42, such that the open end of the housing 36 contacts the body surface 38 of the wrist 42. A pulse wave sensor 46 is supported by the housing 36 via a flexible diaphragm 44, such that the pulse wave sensor 46 is displaceable relative to the housing 36 and is movable out of the housing 36 through its open end. The housing 36, diaphragm 44 and pulse wave sensor 46 cooperate with each other to define a pressure chamber 48, to which pressurized air is supplied from a second air pump 50 via a pressure regulator valve 52. Thus, the pulse wave sensor 46 is pressed against the body surface 38 with a pressing force $P_{HD}$ corresponding to an air pressure in the pressure chamber 48.

The pulse wave sensor 46 includes a plurality of semiconductor pressure-sensing elements (not shown) provided on one of opposite surfaces of a semiconductor substrate consisting of a single crystal of silicon, which one surface serves as a contact surface 54 of the pulse wave sensor 46. The pulse wave sensor 46 is pressed at the contact surface 54 against the body surface 38 of the wrist 42, to detect the oscillatory pressure wave, i.e., pulse wave, which is produced by a radial artery 56 and transmitted to the body surface 38 and the contact surface 54. The pulse wave sensor 46 generates a probe pulse wave signal SM2 representative of the detected pulse wave. The probe pulse wave signal SM2 is supplied to the control device 28 via a third analog to digital (A/D) converter 58.

The CPU 29 of the control device 28 operates according to the control programs stored in the ROM 31, for applying drive signals to the second air pump 50 and the pressure regulator valve 52 so as to adjust the air pressure in the pressure chamber 48, in other words, to adjust the pressing force $P_{HD}$ of the pulse wave sensor 46 which acts on the body surface 38. In monitoring the blood pressure of the patient, the CPU 29 determines an optimum pressing force $P_{HDP}$ of the pulse wave sensor 46, on the basis of the pulse waves obtained while the air pressure in the pressure chamber 48 is continuously changed. The pressure regulator valve 52 is controlled so as to maintain the air pressure in the pressure chamber 48 at an optimum level corresponding to the determined optimum pressing force $P_{HDP}$.

The present monitor system 8 further has a card reader 60 for reading information on a diagnostic information card 62 inserted therein. The card reader 60 is adapted to send the information to the control device 28. In the diagnostic information card 62, there are stored an identification code which identifies a specific living subject or patient (hereinafter referred to as "patient") and blood pressure data obtained during resting of the living subject when the patient received a medical diagnosis before the patient is subjected to blood pressure monitoring operation by the present BP monitor system 8. The memory device 64 is adapted to successively store the physical information obtained while the patient is in a normal condition during operation of the present monitor system 8.

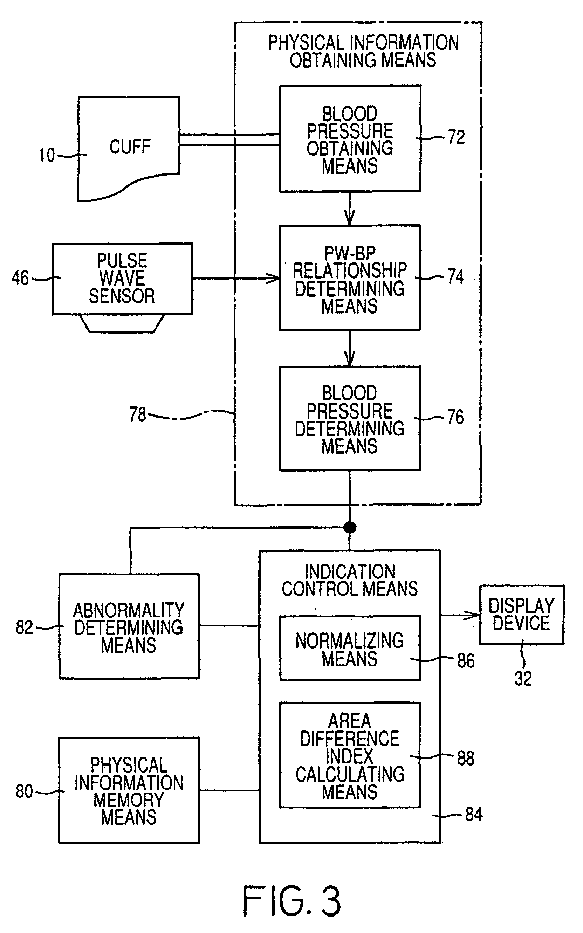

Fig. 3 illustrates various functions of the control device 28 of the present BP monitor system 8, which includes the above-indicated cuff 10, pulse wave sensor 46 and the card reader 60. The BP monitor system 8 further includes physical information obtaining means 78, physical information memory means 80 and abnormality determining means 82. The physical information obtaining means 78 incorporates blood pressure (BP) obtaining means 72, relationship determining means 74 and blood pressure determining means 76. The indication control means 84 incorporates normalizing means 86 and area difference index calculating means 88. The blood pressure (BP) obtaining means 72 is adapted to obtain a systolic blood pressure (SAP) and a diastolic blood pressure (DAP) of the patient according to a known oscillometric method (JIS T 1115). Described in detail, after the pressure in the cuff 10 is first increased up to a predetermined target value (e.g., about 180mmHg) higher by a suitable amount than an expected or estimated systolic blood pressure of the patient, the pressure in the cuff 10 is slowly lowered at a rate of about 3mmHg/sec. The SAP and DAP values are determined on the basis of a change in the magnitudes of successive pulses of the pulse wave obtained by the pulse-wave filter circuit 24 while the pressure in the cuff 10 is slowly lowered. When the blood pressure measurement is completed, the pressure in the cuff 10 is released.

The pulse wave sensor 46 is preferably worn on the wrist 42 of one of the arms 12 of the patient on which the cuff 10 is not wound, so that the pulse wave sensor 46 is pressed against the body surface 38 of the wrist 42 to detect the pulse wave generated from the radial artery of the wrist.

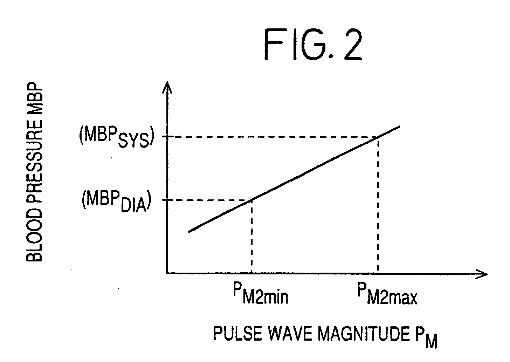

The relationship determining means 74 of the physical information obtaining means 78 is adapted to determine a relationship between a magnitude PM of the pulse wave detected by the pulse wave sensor 46 and the blood pressure determined by the BP obtaining means 72. The relationship between the pulse wave magnitude PM and the blood pressure (hereinafter referred to as "PW-BP relationship") is indicated in a graph of Fig. 2 by way of example, and represented by the following equation:

$$MBP = A \cdot PM + B,$$

wherein    MBP: monitored blood pressure;
           A: a constant which represents a gradient; and
           B: a constant which represents an intercept.

The blood pressure determining means 76 of the physical information obtaining means 78 is adapted to determine a monitored systolic blood pressure $MBP_{SYS}$ and a monitored diastolic blood pressure $MBP_{DIA}$ according to the determined PW-BP relationship, based on the magnitude PM of each pulse wave detected by the pulse wave sensor 46, i.e., on the basis of a maximum pulse wave magnitude (upper peak) $P_{M2max}$ and a minimum pulse wave magnitude (lower peak) $P_{M2min}$. The determined blood pressure values MBP ($MBP_{SYS}$ and $MBP_{DIA}$) are indicated on a display device 32, together with a pressure pulse wave indicative of the blood pressure of the patient, i.e., a waveform of the blood pressure. Thus, the physical information obtaining means 78 is adapted to obtain the blood pressure (BP) waveform as an example of the physical information of the living subject.

The physical information memory means 80 stores, in the memory device 64, the physical information in the form of the BP waveform indicative of the blood pressure of the patient obtained in the comparatively normal condition of the patient after the commencement of operation of the present system 8. The content of the memory device 64 is successively updated. The abnormality determining means 82 is adapted to determine whether the BP waveform is outside a predetermined reference range, e.g., a range of 70mmHg-150mmHg, and generates a signal indicative of abnormality of the blood pressure of the patient when the BP waveform is outside the reference range.

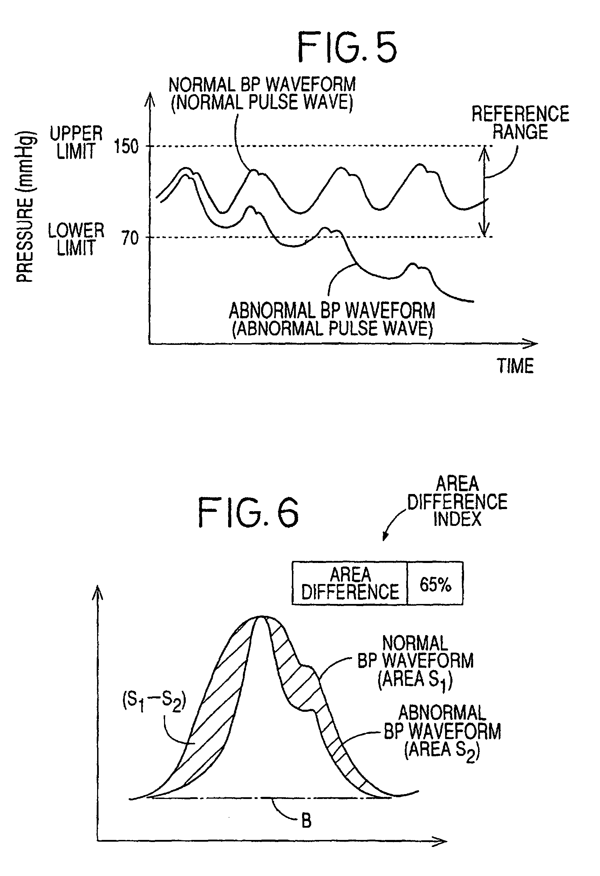

When the abnormality determining means 82 determines that the blood pressure represented by the blood pressure waveform (BP waveform) is abnormal, the indication control means 84 controls a display device 32 to indicate the BP waveform which was determined to be abnormal by the abnormality determining means 82 (abnormal BP waveform), together with the BP waveform (normal BP waveform) obtained in the comparatively normal condition of the patient and stored in the memory means 80, in the same indication area of the display device 32. Fig. 5 is a graph showing an example of an indication provided in the indication area of the display device 32, wherein the waveform of the normal blood pressure (i.e., the normal BP waveform) represented by a series of pulses of a normal pulse wave and the waveform of the abnormal blood pressure (i.e., the abnormal BP waveform) represented by a series of pulses of an abnormal pulse wave are indicated in the same two-dimensional coordinate system in which the time is taken along the abscissa while the magnitude of the pulses, i.e., the blood pressure is taken along the ordinate. It will be understood from the graph of Fig. 5 that the abnormal BP waveform can be compared with the normal BP waveform, so that the degree of abnormality of the blood pressure of the patient, namely, the degree of emergency to deal with the abnormal physical condition of the patient can be easily recognized.

The normalizing means 86 of the indication control means 84 is adapted to normalize the normal BP waveform and the abnormal BP waveform so that the peak amplitude and the wavelength of the abnormal BP waveform coincide with those of the normal BP waveform. The area difference index calculating means 88 is adapted to calculate an area difference index which quantitatively indicates a difference between an area S1 defined by one pulse of the normal BP waveform (i.e., one pulse waveform of the normal pulse wave) and a base line B indicated by a one-dot chain line in the graph of Fig. 5, and an area S2 defined by one pulse of the abnormal BP waveform (i.e., one pulse waveform of the abnormal pulse wave) and the base line B, after the normal and abnormal BP waveforms are normalized by the normalizing means 86. (Hereinafter the area S1 is referred to as "normal pulse waveform area S1" while the area S2 is referred to as "abnormal pulse waveform area S2".) The indication control means 84 controls the display device 32 to indicate the normal pulse waveform and the abnormal pulse waveform such that the two pulse waveforms are superimposed on each other in the same phase, that is, such that the reference points of the two pulse waveforms are aligned with each other in the directions of the amplitude and wavelength. The display device 32 also indicates the area difference index calculated by the calculating means 88. The area difference index may be represented as: (S1 - S2) which is a difference between the normal pulse waveform area S1 and the abnormal pulse waveform area S2; S2/S1 (%) which is a ratio of the abnormal pulse waveform area S2 to the normal pulse waveform area S1; (S1 - S2)/S1 (%) which is a ratio of the area difference (S2 - S1) to the normal pulse waveform area S1. In Fig. 6, the area difference index S2/S1 (%) is indicated.

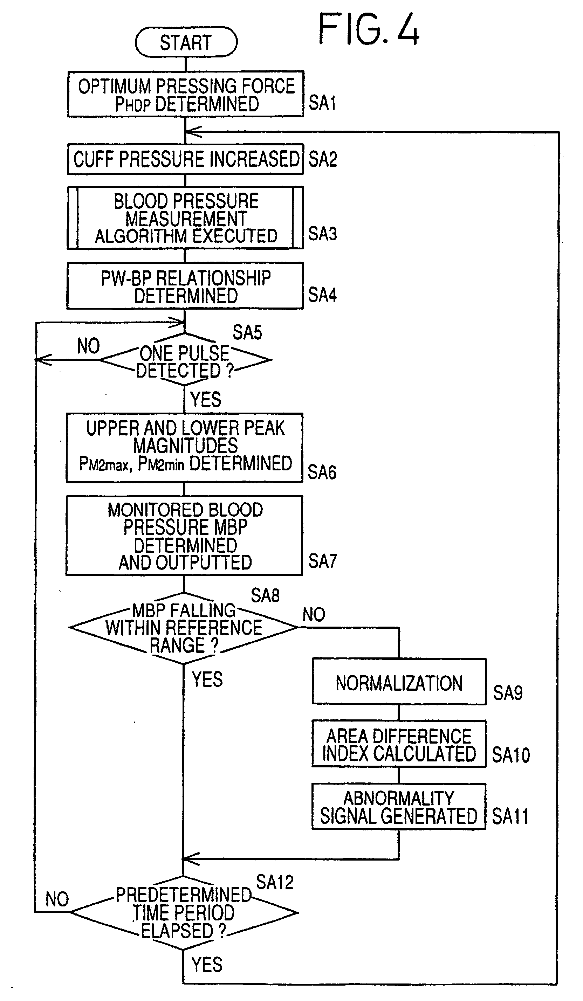

There will be described the operation of the control device 28 referring to the flow chart of Fig. 4.

A control routine illustrated in the flow chart of Fig. 4 is initiated with step SA1 in which the air pressure in the pressure chamber 48 is slowly raised, and the CPU 29 determines the optimum air pressure in the pressure chamber 48, at which the amplitude of a pulse detected by the pulse wave sensor 46 is maximized. The air pressure in the chamber 48 is held at the determined optimum level so that the pressing force of the pulse wave sensor 46 is maintained at the optimum value $P_{HDP}$.

Step SA1 is followed by step SA2 in which the pressure in the cuff 10 is raised for effecting the blood pressure measurement in the following step SA3 corresponding to the BP obtaining means 72. In this step SA3, the blood pressure of the patient is measured according to a predetermined blood pressure measuring algorithm. Described in detail, following the increase of the cuff pressure in step SA2, the selector valve 16 is placed in the inflation position and the first air pump 18 is actuated so as to increase the pressure in the cuff 10 up to a target value (e.g., 180mmHg) higher by a suitable amount than the estimated systolic blood pressure of the patient. Subsequently, the first air pump 18 is turned off and the selector valve 16 is switched from the inflation position to the slow-deflation position so as to slowly decrease the pressure in the cuff 10 at a rate of 3mmHg/sec. The systolic blood pressure (SAP), mean blood pressure (MAP) and diastolic blood pressure (DAP) are determined based on variation of the amplitudes of successive pulses of the cuff pulse wave signal SM1 obtained during the slow decreasing of the cuff pressure, according to a well-known oscillometric blood pressure determining algorithm, for example. Further, the pulse rate (PR) is determined based on an interval between successive adjacent pulses of the cuff pulse wave signal SM1. The determined SAP, MAP, DAP and PR values are indicated on the display 32, and the selector valve 16 is switched from the slow-deflation position to the rapid-deflation position, whereby the pressure in the cuff 10 is rapidly lowered.

The control flow then goes to step SA4 corresponding to the PW-BP relationship determining means 74 so as to obtain a relationship between the magnitude (absolute value) of each pulse of the pulse wave detected by the pulse wave sensor 46 (i.e., magnitude of the probe pulse wave signal SM2) and the blood pressure values SAP, DAP measured by using the cuff 10 in step SA3. In other words, one pulse of the probe pulse wave signal SM2 from the pulse wave sensor 46 is read to determine a maximum and a minimum magnitude $P_{M2max}$, $P_{M2min}$ of that pulse. Subsequently, the PW-BP relationship determining means 74 determines the relationship between the pulse wave magnitude PM and the monitored blood pressure MBP as indicated in the graph of Fig. 2, on the basis of the determined maximum and minimum pulse wave magnitudes $P_{M2max}$, $P_{M2min}$, and the systolic and diastolic blood pressure values SAP, DAP obtained by using the cuff 10 in step SA3.

Step SA4 is followed by step SA5 to determine whether one pulse of the probe pulse wave signal SM2 has been supplied from the pulse wave sensor 46. As long as a negative decision is obtained in step SA5, this step is repeatedly implemented. If an affirmative decision is obtained in step SA5, the control flow goes to steps SA6 and SA7 corresponding to the blood pressure determining means 76, so as to determine the maximum and minimum magnitudes (upper and lower peaks) of the pulse of the probe pulse wave signal SM2 received from the pulse wave sensor 46 which is pressed against the body surface 38 with the above-described optimum pressing force $P_{HDP}$. Further, a monitored systolic blood pressure $MBP_{SYS}$ and a monitored diastolic blood pressure $MBP_{DIA}$ are determined based on the maximum and minimum magnitudes $P_{M2max}$ and $P_{M2min}$ of the pulse of the probe pulse wave signal SM2, according to the PW-BP relationship (as shown in the graph of Fig. 2) which has been obtained in step SA4 as described above. The determined monitored systolic and diastolic blood pressure values $MBP_{SYS}$ and $MBP_{DIA}$ are indicated on the display device 32, together with a series of pulses of the pulse wave calibraled or determined according to the above-described PW-BP relationship, i. e., together with a blood pressure (BP) waveform.

The control flow then goes to step SA8 corresponding to the abnormality determining means 82 to determine whether the monitored blood pressure determined in step SA7 falls within the predetermined reference range. The reference range used for determining whether the blood pressure of the living subject is abnormal is suitably determined by the upper limit (systolic blood pressure $MBP_{SYS}$) of 150mmHg and the lower limit (diastolic blood pressure $MBP_{DIA}$) of 70mmHg.

If a negative decision is obtained in step SA8, this means that the blood pressure of the patient is abnormal, and the control flow goes to step SA9 corresponding to the normalizing means 86 so as to implement the normalization for superimposing the abnormal pulse wave and the normal pulse wave on each other such that the reference points of the abnormal and normal pulse waves are aligned with each other in the directions of the amplitude and wavelength. Then, the control flow goes to step SA10 corresponding to the area difference index calculating means 88 to calculate, as the area difference index, either one of: (S1 - S2) which is a difference between the normal pulse waveform area S1 and the abnormal pulse waveform area S2; S2/S1 (%) which is a ratio of the abnormal pulse waveform area to the normal pulse waveform area; and (S1 - S2)/S1 (%) which is a ratio of the area difference (S1 - S2) to the normal pulse waveform area S1.

Step SA10 is followed by step SA11 to generate the abnormality signal indicative of the abnormal blood pressure of the patient. The abnormality is visually indicated on the display device 32, for instance, by a flickering, colored or enlarged area of the display screen. Alternatively, the abnormality is indicated by an audible alarm device adapted to generate a sound or voice, or by other devices adapted to inform the abnormal blood pressure of the patient. The display device 32 is controlled to indicate the normal pulse wave and the abnormal pulse wave in the same coordinate system as shown in Figs. 5 and 6, together with the area difference index calculated by the calculating means 88.

Step SA11 is followed by step SA12. This step SA12 is effected immediately following step SA8 if an affirmative decision is obtained in step SA8. Step SA12 is provided to determine whether a predetermined time period has elapsed after the commencement of the blood pressure measurement by using the cuff 10 in step SA3. For example, the predetermined time period is several tens of minutes. If a negative decision is obtained in step SA12, the control flow goes back to step SA5 and the following steps for determining the monitored systolic and diastolic blood pressure $MBP_{SYS}$ and $MBP_{DIA}$ of the pulse wave signal SM2 and indicating the determined blood pressures $MBP_{SYS}$ and $MBP_{DIA}$ on the display device 32. On the other hand, if an affirmative decision is obtained in step SA12, the control flow goes back to step SA2 and the following steps so as to update the PW-BP relationship.

According to the present embodiment described above, the memory device 64 corresponding to the physical information memory means 80 stores the BP waveform indicative of the blood pressure obtained in a comparatively normal condition of the patient. When the abnormality determining means 82 corresponding to step SA8 determines that the BP waveform obtained during the current monitoring operation is outside the reference range, the indication control means 84 corresponding to steps SA9 and SA10 controls the display device 32 to indicate the BP waveform which is determined to be abnormal by the abnormality determining means 88 (abnormal BP waveform), and the BP waveform (normal BP waveform) which was obtained in the comparatively normal condition and stored in the memory device 64, in the same indication area of the display device 32. This arrangement permits the medical workers to easily recognize the degree of abnormality of the blood pressure of the patient, i.e., the degree of emergency to deal with the abnormal physical condition of the patient, simply by comparing the normal BP waveform and the abnormal BP waveform which are indicated in the same display area.

According to the present embodiment, the indication control means 84 controls the display device 32 to indicate a series of pulses of the normal pulse wave (the normal BP waveform indicative of the normal blood pressure of the patient) and a series of pulses of the abnormal pulse wave (the abnormal BP waveform indicative of the abnormal blood pressure of the patient), in the same two-dimensional coordinate system in which the time is taken along the abscissa while the magnitude of the pulses, i.e., the blood pressure is taken along the ordinate. This arrangement assures accurate comparison between the magnitude or amplitude of the normal pulse wave (the pressure difference between the systolic blood pressure and the diastolic blood pressure), i. e., pulse pressure, and the magnitude or amplitude of the abnormal pulse wave.

The indication control means 84 further controls the display device 32 to indicate one pulse waveform of the normal pulse wave (normal BP waveform) and one pulse waveform of the abnormal pulse wave (abnormal BP waveform), such that the phases of the two pulse waveforms coincide with each other as shown in the graph of Fig. 6. This arrangement permits easier comparison between the normal and abnormal pulse waveforms.

The indication control means 84 includes the normalizing means 86 for normalizing the normal and abnormal pulse waves such that at least one of the amplitude and wavelength of the abnormal pulse wave coincide with the corresponding one of the amplitude and wavelength of the normal pulse wave. After the normal and abnormal pulses are normalized by the normalizing means 86, the display device 32 indicates one pulse waveform of the normal pulse wave and the corresponding pulse waveform of the abnormal pulse wave, such that the normal and abnormal pulse waveforms are superimposed on each other. Thus, the difference between the configurations of the normal and abnormal pulse waveforms can be easily recognized.

The indication control means 84 further includes the area difference index calculating means 88 for calculating the area difference index which numerically indicates the difference between the area defined by the above-indicated one normal pulse waveform and the area defined by the above-indicated one abnormal pulse waveform, after the pulses are normalized by the normalizing means 86. The calculated area difference index is also graphically indicated on the display device 32, so that the difference between the configurations of the normal and abnormal pulse waveforms can be quantitatively recognized.

While the present invention has been described in its presently proffered embodiment, it is to be understood that the invention may be otherwise modified.

In the illustrated embodiment, a series of pulses of the normal pulse wave and a series of pulses of the abnormal pulse wave are indicated in the same coordinate system as shown in Fig. 5, and one normal pulse waveform and one abnormal pulse waveform which are superimposed on each other are indicated in the same coordinate system as shown in Fig. 6, so that the normal and abnormal pulse waves can be easily compared with each other. However, the advantage of the present invention can be similarly attained when the display device 32 is adapted to provide only one of the graphical representations as shown in Figs. 5 and 6. If the display device 32 is adapted to provide only the graphical representation of Fig. 5, the normalizing means 86 and the area difference index calculating means 88 may be eliminated.

The reference range used in step SA8 for determining the abnormality of the blood pressure may be automatically determined on the basis of the blood pressure obtained in a comparatively normal condition of the patient. The determination of the abnormality of the blood pressure may be effected when either one of the systolic blood pressure (SAP), mean blood pressure (MAP) and diastolic blood pressure (DAP) is outside the corresponding reference range.

In the illustrated embodiment, the blood pressure is monitored as the physical information. However, the blood pressure may be replaced with blood oxygen saturation, temperature, pulse rate or fluctuation of these parameters (in a given time period).

The blood pressure obtaining means 72 is adapted to determine the blood pressure according to the known oscillometric method, based on a change in the magnitude of the pulses of the pulse wave, which change is detected as the pressure in the cuff 10 is varied. However, the blood pressure obtaining means 72 may be adapted to determine the blood pressure according to the known Korotkoff-sound method in which a microphone is used to detect presence and absence of Korotkoff sounds of an artery as the pressure in the cuff 10 is changed.

It is to be understood that the present invention may be embodied with other changes, improvements and modifications which will occur to those skilled in the art without departing from the scope of the invention defined in the appended claims.

**Claims**

1. A physical information monitor system, comprising:

> physical information obtaining means (78) for successively obtaining physical information of a living subject;
> physical information memory means (80) for storing said physical information which is obtained by said physical information obtaining means and which is within a predetermined reference range;
> abnormality determining means (82) for determining that said physical information obtained by said physical information obtaining means is abnormal if said physical information is outside said predetermined reference range;
> a display device (32) for indicating said physical information obtained by said physical information obtaining means; and
indication control means (84) for controlling said display device to indicate, in a same indication area of said display device, said physical information which has been determined to be abnormal by said abnormality determining means and said physical information previously stored by said physical information memory means, said system being **characterized in that**:

> said physical information obtaining means obtains as said physical information a series of pulses of a pulse wave generated in synchronism with heartbeat of said subject, while said indication control means controls said display device to indicate, in a same two-dimensional coordinate system, a waveform of one of a series of pulses of a normal pulse wave obtained by said physical information obtaining means in a normal condition of said subject and a waveform of one of a series of pulses of an abnormal pulse wave obtained by said physical information obtaining means in an abnormal condition of said subject, such that phases of said waveforms of said normal pulse wave and said abnormal pulse wave coincide with each other, in said same two-dimensional coordinate system in which time is taken along an abscissa while an amplitude of said series of pulses is taken along an ordinate.

2. A physical information monitor system according to claim 1, wherein said indication control means controls said display device to indicate said series of pulses of said normal pulse wave obtained by said physical information obtaining means in said normal condition of said subject and said series of pulses of said abnormal pulse wave obtained by said physical information obtaining means in said abnormal condition of said subject, in a same two-dimensional coordinate system in which time is taken along an abscissa while an amplitude of said series of pulses is taken along an ordinate.

3. A physical information monitor system according to claim 1, wherein said indication control means includes normalizing means (86) for normalizing said waveforms of said normal pulse wave and said abnormal pulse wave, such that at least one of an amplitude and a wavelength of said abnormal pulse wave coincides with the corresponding one of the amplitude and wavelength of said normal pulse wave, said indication control means controlling said display device to indicate the waveforms of said normal pulse wave and said abnormal pulse wave which have been normalized by said normalizing means, such that said waveforms of said normal pulse wave and said abnormal pulse wave are superimposed on each other.

4. A physical information monitor system according to claim 3, wherein said indication control means includes calculating means (88) for calculating an area difference index which quantitatively indicates a difference between an area defined by said waveform of said normal pulse wave and an area defined by said waveform of said abnormal pulse wave, said indication control means controlling said display device to indicate said area difference index calculated by said calculating means.

5. A physical information monitor system according to any one of claims 1-4, wherein said physical information obtaining means comprises:

pulse wave detecting means (46) for detecting said series of pulses of said pulse wave generated in synchronism with heartbeat of said subject;

blood pressure obtaining means (72) including an inflatable cuff (10), for obtaining a blood pressure of said living subject;

relationship determining means (74) for determining a relationship between a magnitude of said pulse wave detected by said pulse wave detecting means and said blood pressure obtained by said blood pressure obtaining means; and

blood pressure determining means (76) for determining a monitored systolic blood pressure ($MBP_{SYS}$) and a monitored diastolic blood pressure ($MBP_{DIA}$) based on said magnitude of said pulse wave and said relationship determined by said determining means.

6. A physical information monitor system according to any one of claims 1-5, wherein said physical information comprises blood pressure of said living subject.

**Patentansprüche**

1. Überwachungssystem für physische Informationen, umfassend:

Erhaltungsmittel für physische Informationen (78) zum fortlaufenden Erhalten einer physischen Information eines lebenden Subjektes;

Speichermittel für physische Informationen (80) zum Speichern der physischen Information, welche durch das Erhaltungsmittel für physische Informationen erhalten wird und welche innerhalb eines vorbestimmten Referenzbereichs liegt;

Abnormitätsbestimmungsmittel (82) zum Bestimmen, daß die physische Information, die durch das Erhaltungsmittel für physische Informationen erhalten wurde, abnormal ist, wenn die physische Information außerhalb des vorbestimmten Referenzbereichs liegt;

eine Anzeigeeinrichtung (32) zum Anzeigen der physischen Information, die durch das Erhaltungsmittel für physische Informationen erhalten wurde; und

Anzeigesteuermittel (84) zum Steuern der Anzeigeeinrichtung, um in einem gleichen Anzeigebereich der Anzeigeeinrichtung die physische Information, die durch das Abnormitätsbestimmungsmittel als abnormal bestimmt wurde, und die physische Information, die vorher durch das Speichermittel für physische Informationen gespeichert wurde, anzuzeigen, wobei das System dadurch charakterisiert ist, daß:

das Erhaltungsmittel für physische Informationen als die physische Information eine Serie von Pulsen einer Pulswelle, die synchron mit dem Herzschlag des Subjektes generiert wird, erhält, während das Anzeigesteuermittel die Anzeigeeinrichtung steuert, um in einem gleichen zweidimensionalen Koordinatensystem eine Wellenform von einer aus einer Serie an Pulsen einer normalen Pulswelle, die durch das Erhaltungsmittel für physische Informationen in einem normalen Zustand des Subjektes erhalten wurde, und eine Wellenform von einer aus einer Serie von Pulsen einer abnormalen Pulswelle, die durch das Erhaltungsmittel für physische Informationen in einem abnormalen Zustand des Subjektes erhalten wurde, anzuzeigen, so daß Phasen der Wellenformen der normalen Pulswelle und der abnormalen Pulswelle miteinander in demselben zweidimensionalen Koordinatensystem koinzidieren, in welchem die Zeit entlang der Abszisse aufgetragen wird, während eine Amplitude der Serien an Pulsen entlang der Ordinate aufgetragen wird.

2. Ein Überwachungssystem für physische Informationen gemäß Anspruch 1, wobei das Anzeigesteuermittel die Anzeigeeinrichtung steuert, um die Serie an Pulsen der normalen Pulswelle, die durch das Erhaltungsmittel für physische Informationen in dem normalen Zustand des Subjektes erhalten wurde, und die Serie an Pulsen der abnormalen Pulswelle, die durch das Erhaltungsmittel für physische Informationen in dem abnormalen Zustand des Subjektes erhalten wurde, in einem gleichen zweidimensionalen Koordinatensystem anzuzeigen, in welchem die Zeit entlang der Abszisse aufgetragen wird, während eine Amplitude der Serie an Pulsen entlang der Ordinate aufgetragen wird.

3. Ein Überwachungssystem für physische Informationen gemäß Anspruch 1, wobei das Anzeigesteuermittel ein Normalisierungsmittel (86) zum Normalisieren der Wellenformen der normalen Pulswelle und der abnormalen Pulswelle enthält, so daß wenigstens eine von einer Amplitude und einer Wellenlänge der abnormalen Pulswelle mit der korrespondierenden der Amplitude und Wellenlänge der normalen Pulswelle koinzidiert, wobei das Anzeigesteuermittel die Anzeigeeinrichtung steuert, um die Wellenformen der normalen Pulswelle und der abnormalen Pulswelle, welche durch das Normalisierungsmittel normalisiert wurden, so anzuzeigen, daß die Wellenformen der normalen Pulswelle und der abnormalen Pulswelle übereinander liegen.

4. Ein Überwachungssystem für physische Informationen gemäß Anspruch 3, wobei das Anzeigesteuermittel ein Berechnungsmittel (88) enthält zum Berechnen eines Flächenunterschiedindexes, welcher quantitativ einen Unterschied zwischen einer Fläche, die durch die Wellenform der normalen Pulswelle definiert ist, und einer Fläche, die durch die Wellenform der abnormalen Pulswelle definiert ist, anzeigt, wobei das Anzeigesteuermittel die Anzeigeeinrichtung steuert, um den Flächenunterschiedindex, der durch das Berechnungsmittel errechnet wurde, anzuzeigen.

5. Ein Überwachungssystem für physische Informationen gemäß einem der Ansprüche 1-4, wobei das Erhaltungsmittel für physische Informationen folgendes umfaßt:

Pulswellenerfassungsmittel (46) zum Erfassen der Serie von Pulsen der Pulswelle, die synchron mit dem Herzschlag des Subjektes generiert wird;

Blutdruckerhaltungsmittel (72) einschließlich einer aufblasbaren Manschette (10) zum Erhalten eines Blutdruckes des lebenden Subjektes;

Verhältnisbestimmungsmittel (74) zum Bestimmen eines Verhältnisses zwischen einer Größe der Pulswelle, die durch das Pulswellenerfassungsmittel erfaßt wurde, und des Blutdrucks, der durch das Blutdruckerhaltungsmittel erhalten wurde; und

Blutdruckbestimmungsmittel (76) zum Bestimmen eines überwachten systolischen Blutdrucks (MBP$_{SYS}$) und eines überwachten diastolischen Blutdrucks (MBP$_{DIA}$), basierend auf der Größe der Pulswelle und des Verhältnisses, das durch das Bestimmungsmittel bestimmt wurde.

6. Ein Überwachungssystem für physische Informationen gemäß einem der Ansprüche 1-5, wobei die physische Information den Blutdruck eines lebenden Subjektes umfaßt.

## Revendications

1. Système de surveillance d'informations physiques, comprenant :

des moyens d'obtention d'informations physiques (78) pour obtenir successivement des informations physiques sur un sujet vivant ;
des moyens de mémoire d'informations physiques (80) pour stocker lesdites informations physiques qui sont obtenues par lesdits moyens d'obtention d'informations physiques et qui sont à l'intérieur d'une plage de référence prédéterminée ;
des moyens de détermination d'anomalies (82) pour déterminer que lesdites informations physiques obtenues par lesdits moyens d'obtention d'informations physiques sont anormales si lesdites informations physiques sont en dehors de ladite plage de référence prédéterminée ;
un dispositif d'affichage (32) pour indiquer lesdites informations physiques obtenues par lesdits moyens d'obtention d'informations physiques ; et
des moyens de contrôle d'indication (84) pour contrôler ledit dispositif d'affichage pour indiquer, dans une même zone d'indication dudit dispositif d'affichage, lesdites informations physiques qui ont été déterminées comme étant anormales par lesdits moyens de détermination d'anomalies et lesdites informations physiques précédemment stockées par lesdits moyens de mémoire d'informations physiques, ledit système étant **caractérisé en ce que** :

lesdits moyens d'obtention d'informations physiques obtiennent comme informations physiques une série de pulsations d'une onde de pouls générée en synchronisme avec le battement de coeur dudit sujet, pendant que lesdits moyens de contrôle d'indication contrôlent ledit dispositif d'affichage pour indiquer, dans un même système de coordonnées bidimensionnel, la forme d'onde de l'une d'une série de pulsations d'une onde de pouls normale obtenue par lesdits moyens d'obtention d'informations physiques dans un état normal dudit sujet et la forme d'onde de l'une d'une série de pulsations d'une onde de pouls anormale obtenue par lesdits moyens d'obtention d'informations physiques dans un état anormal dudit sujet, de sorte que des phases desdites formes d'onde de ladite onde de pouls normale et de ladite onde de pouls anormale coïncident l'une avec l'autre, dans ledit même système de coordonnées bidimensionnel dans lequel le temps est porté sur un axe des abscisses tandis que l'amplitude de ladite série de pulsations est portée sur un axe des ordonnées.

2. Système de surveillance d'informations physiques selon la revendication 1, dans lequel lesdits moyens de contrôle d'indication contrôlent ledit dispositif d'affichage pour indiquer ladite série de pulsations de ladite forme d'onde normale obtenue par lesdits moyens d'obtention d'informations physiques dans ledit état normal dudit sujet et ladite série de pulsations de ladite forme d'onde anormale obtenue par lesdits moyens d'obtention d'informations physiques dans ledit état anormal dudit sujet, dans un même système de coordonnées bidimensionnel dans lequel le temps est porté sur un axe des abscisses tandis que l'amplitude de ladite série de pulsations est portée sur un axe des ordonnées.

3. Système de surveillance d'informations physiques selon la revendication 1, dans lequel lesdits moyens de contrôle d'indication comprennent des moyens de normalisation (86) pour normaliser lesdites formes d'onde de ladite onde de pouls normale et de ladite onde de pouls anormale, de sorte qu'au moins une amplitude et une longueur d'onde de ladite onde de pouls anormale coïncident avec une amplitude et une longueur d'onde correspondante de ladite onde de pouls normale, lesdits moyens de contrôle d'indication contrôlant ledit dispositif d'affichage pour indiquer les formes d'onde de ladite onde de pouls normale et de ladite onde de pouls anormale qui ont été normalisées par lesdits moyens de normalisation, de sorte que lesdites formes d'onde de ladite onde de pouls normale et de ladite onde de pouls anormale sont superposées.

4. Système de surveillance d'informations physiques selon la revendication.3, dans lequel lesdits moyens de contrôle d'indication comprennent des moyens de calcul (88) pour calculer un indice différentiel de zone qui indique une différence quantitative entre une zone définie par ladite forme d'onde de ladite onde de pouls normale et une zone définie par ladite forme d'onde de ladite onde de pouls anormale, lesdits moyens de contrôle d'indication contrôlant ledit dispositif d'affichage pour indiquer ledit indice différentiel de zone calculé par lesdits moyens de calcul.

5. Système de surveillance d'informations physiques selon l'une quelconque des revendications 1 à 4, dans lequel lesdits moyens d'obtention d'informations physiques comprennent :

des moyens de détection d'onde de pouls (46) pour détecter lesdites séries de pulsations de ladite onde de pouls générée en synchronisme avec le battement de coeur dudit sujet ;
des moyens d'obtention de pression artérielle (72) comprenant une manchette gonflable (10), pour obtenir une pression artérielle dudit sujet vivant ;
des moyens de détermination de relation (74) pour déterminer une relation entre l'importance de ladite onde de pouls détectée par lesdits moyens de détection d'onde de pouls et ladite pression artérielle obtenue par lesdits moyens d'obtention de pression artérielle ; et
des moyens de détermination de pression artérielle (76) pour déterminer une pression artérielle systolique surveillée ($MBP_{SYS}$) et une pression artérielle diastolique surveillée ($MBP_{DIA}$) à partie de ladite importance de ladite onde de pouls et de ladite relation déterminée par lesdits moyens de détermination.

6. Système de surveillance d'informations physiques selon l'une quelconque des revendications 1 à 5, dans lequel lesdites informations physiques comprennent la pression artérielle dudit sujet vivant.

FIG.1

# FIG. 2

FIG.3

# FIG. 4

```
                    START

        OPTIMUM PRESSING FORCE      SA1
          PHDP DETERMINED

        CUFF PRESSURE INCREASED     SA2

          BLOOD PRESSURE
           MEASUREMENT             SA3
        ALGORITHM EXECUTED

        PW-BP RELATIONSHIP
           DETERMINED              SA4

                              SA5
    NO        ONE PULSE
        ◄──   DETECTED ?
                 YES

        UPPER AND LOWER PEAK
            MAGNITUDES
      PM2max, PM2min DETERMINED    SA6

        MONITORED BLOOD
         PRESSURE MBP
          DETERMINED
        AND OUTPUTTED              SA7

                          SA8
          MBP FALLING          NO
        WITHIN REFERENCE      ──────►
           RANGE ?
             YES                    NORMALIZATION      SA9

                                  AREA DIFFERENCE
                                  INDEX CALCULATED    SA10

                                   ABNORMALITY
                                 SIGNAL GENERATED     SA11

                            SA12
    NO      PREDETERMINED
        ──  TIME PERIOD
             ELAPSED ?
               YES
```

# FIG. 5

NORMAL BP WAVEFORM
(NORMAL PULSE WAVE)

REFERENCE
RANGE

PRESSURE (mmHg)

UPPER
LIMIT    150

LOWER
LIMIT    70

ABNORMAL BP WAVEFORM
(ABNORMAL PULSE WAVE)

TIME

# FIG. 6

AREA
DIFFERENCE
INDEX

| AREA DIFFERENCE | 65% |
|---|---|

NORMAL
BP WAVEFORM
(AREA $S_1$)

ABNORMAL
BP WAVEFORM
(AREA $S_2$)

$(S_1 - S_2)$

B